# EUROPEAN PATENT APPLICATION

(11) **EP 4 696 262 A1**
(43) Date of publication of application: **18.02.2026**
(21) Application number: 24933197.6
(22) Date of filing: 26.07.2024
(51) Int. Cl.: A61B 18/12, A61N 5/00

(54) **RADIO FREQUENCY ELECTRODE ARRAY CONTROL DEVICE AND RADIO FREQUENCY THERAPY INSTRUMENT**

(30) Priority: 31.05.2024 CN 202410700787
(71) Applicant: Shenzhen Peninsula Medical Group, Shenzhen, Guangdong 518000 (CN)
(72) Inventor: LIANG, Yongsheng, Shenzhen, Guangdong 518000 (CN); LEI, Xiaobing, Shenzhen, Guangdong 518000 (CN)
(74) Representative: Osterhoff, Utz
(86) International application number: PCT/CN2024/107876
(87) International publication number: WO 2025/246004

(57) **Abstract**

Disclosed are a radio frequency (RF) electrode array control device and an RF treatment equipment, which relate to the field of cosmetic medicine. The RF electrode array control device is applied to the RF treatment equipment, which includes an RF electrode array, and the RF electrode array includes a plurality of RF electrode subarrays. The device includes: a subarray determination module, configured to determine a target time sequence based on the RF electrode array, the target time sequence at least including a Nth subarray and a (N+n)th subarray activated in sequential timings, N and n are both positive integers; and a control module, configured to control the RF electrode subarrays in the target time sequence to be individually activated to output RF energy, and control the Nth subarray and the (N+n)th subarray simultaneously output RF energy in an overlapping period between an output period of the Nth subarray and the output period of the (N+n)th subarray.

## Description

### CROSS-REFERENCE TO RELATED APPLICATIONS

The present application claims priority to Chinese Patent Application No. 202410700787.6, filed on May 31, 2024, and the entire contents of which are incorporated herein by reference.

### TECHNICAL FIELD

The present application relates to the technical field of cosmetic medical technology, and in particular to a radio frequency electrode array control device and a radio frequency (RF) treatment equipment.

### BACKGROUND

The radio frequency (RF) electrode array is a skin treatment technology that an electrode array delivers RF energy inside the skin for aesthetic treatment. During the entire operation of the RF electrodes, the primary source of pain experienced by the human body comes from the process of the RF electrodes delivering RF energy inside the skin, rather than from the process of the RF electrodes penetrating the skin. This sensation of pain is one of the main factors affecting user experience. The pain generated during the process of RF energy delivered by the electrodes is mainly correlated with factors such as the duration of action, the area of action at the same time, and the power of the RF energy.

RF electrode arrays are classified into minimally invasive and noninvasive types based on whether there is a physical needle available for penetrating the skin to directly deliver high frequency energy into skin dermis (between epidermis and subcutaneous layer). The minimally invasive RF electrode array includes an array of RF microneedles. In the prior art, the microneedles coupled with a RF power supply serve as electrodes of delivering the RF energy into the skin tissue, and after penetrating the skin surface, the array of RF microneedles as a whole are simultaneously provided with a preset voltage, causing the RF microneedles to release RF current into the tissue to be treated until the releasing is completed. This method causes the treatment area being covered with the array of microneedles in full size. To achieve the treatment effect with providing a preset total RF power to the tissue, the treatment time must be prolonged to accumulate RF energy to a degree of the preset total RF power, therefore the treatment time on the tissue is longer to cause bigger pain for the patients. For noninvasive RF electrode arrays, although there is no pain from the repeated penetration, to achieve the desired treatment effect, the noninvasive RF electrode arrays often deliver the RF energy with bigger output power of the RF energy. The side effects due to the longer treatment time and bigger pain reaction still exist in patients.

Therefore, given that the total output power of the RF energy is constant in the prior art, the treatment area being covered with the array of microneedles in full size in any moment, resulting in a larger instantaneous treatment size, as well as the longer treatment time, which may cause bigger pain reaction in patients.

### SUMMARY

The main purpose of the present application is to provide a radio frequency (RF) electrode array control device and an RF treatment equipment, aiming to solve the technical problem in the related art that the radio frequency electrode array has a larger instantaneous treatment size and a longer treatment time.

In order to achieve the above purpose, the present application adopts the following technical solutions.

In a first aspect, the present application provides an RF electrode array control device, applied to an RF treatment equipment, and the RF treatment equipment includes an RF electrode array, the RF electrode array includes a plurality of RF electrode subarrays, and each RF electrode subarray includes at least one RF electrode, including: a subarray determination module, configured to select a target sequence according to the RF electrode array, the target sequence including at least one Nth subarray and one (N+n)th subarray activated in sequential timings, N and n are both positive integers; and a control module, configured to control the RF electrode subarrays in the target time sequence to be individually activated to output RF energy, and control the Nth subarray and the (N+n)th subarray simultaneously output RF energy in an overlapping period between an output period of the Nth subarray and the output period of the (N+n)th subarray.

In an embodiment of the present application, in response to determining n is one, the Nth subarray and the (N+n)th subarray are successively activated in the target time sequence.

In an embodiment of the present application, in an overall time period during which the RF electrode subarrays are controlled to be individually activated, the output period of each RF electrode subarray is continuous without interruption.

In an embodiment of the present application, at least part of the output periods of the RF electrode subarrays in the target time sequence includes a first time period during which only one RF electrode subarray is activated to output RF energy.

In an embodiment of the present application, in an overall time period during which the RF electrode subarrays are controlled to be individually activated, the output period of the (N+n)th subarray includes at least two noncontinuous second time periods during which the (N+n)th subarray is activated to output RF energy.

In an embodiment of the present application, the subarray determination module includes:
a first subarray determination submodule, configured to determine a first RF electrode subarray is activated to output RF energy, and store the first RF electrode subarray as a target RF electrode subarray in an initial sequence;
a second subarray determination submodule, configured to determine a new target RF electrode subarray from the remaining RF electrode subarrays in the RF electrode array excluding the target RF electrode subarrays, and store the new target RF electrode subarray in the initial sequence; and
a target sequence generation submodule, configured to repeat executing the second subarray determination submodule to obtain the target time sequence until all RF electrode subarrays in the RF electrode array are the target RF electrode subarrays.

In an embodiment of the present application, in any of the overlapping periods between the output periods of the Nth subarray and the output period of the (N+n)th subarray, the Nth subarrays and the (N+n)th subarray are not physically adjacent with each other.

In an embodiment of the present application, the second subarray determination submodule is configured to randomly select the RF electrode subarray from the remaining RF electrode subarrays in the RF electrode array excluding the target RF electrode subarrays as a new target RF electrode subarray, and store the new target RF electrode subarray in the initial sequence.

In an embodiment of the present application, the RF electrode array control device further includes:
an output accumulating module, configured to accumulate the RF energy outputted by each of the RF electrode subarray activated from firstly to currently; and
when activating each of the RF electrode subarrays, in response to determining the accumulated RF energy of a certain RF electrode subarray is greater than or equal to a preset threshold, and the control module further controls the RF electrode subarray to stop outputting the RF energy.

In an embodiment of the present application, the control module further includes:
a temperature parameter acquisition unit, configured to acquire a real-time temperature parameter collected by a temperature sensor; and
an output power adjustment unit, configured to adjust a real-time output power of an RF electrode subarray currently outputting RF energy according to the real-time temperature parameter.

In an embodiment of the present application, each of the RF electrode subarrays includes an identical number of RF electrodes, or a difference between numbers of the RF electrodes of any two RF electrode subarrays is less than a preset number threshold.

The present application further provides the RF treatment equipment, which includes: an RF power supply; an RF electrode array; and the RF electrode array control device as described above, the RF power supply and the RF electrode array control device are both connected to the RF electrode array.

In an embodiment of the present application, each of the RF electrode subarrays is connected in parallel and connected to the RF power supply, and the RF power supply generates unchanged output power during the overlapping period between the output periods of the Nth subarray and the (N+n)th subarray.

In an embodiment of the present application, the RF power supply includes a plurality of sub-RF power supplies, each of which is connected with each of the RF electrode subarrays to allow an output power of each RF electrode subarray to remain unchanged.

In an embodiment of the present application, the RF treatment equipment includes: a monopolar mode, polarities of RF electrodes of any one RF electrode subarray are same, while an electrode plate of the RF treatment equipment has an opposite polarity.

In an embodiment of the present application, the RF treatment equipment includes: a bipolar mode; each of the RF electrode subarrays includes at least two RF electrodes with opposite polarities in the bipolar mode.

The above one or more technical solutions provided by this application may have the following advantages or at least achieve the following technical effects:

The embodiment of the present application provides an RF electrode array control device and an RF treatment equipment. The RF electrode array control device includes: a subarray determination module and a control module. The target sequence is generated according to the plurality of RF electrode subarrays of the RF electrode array by the subarray determination module, and each of the RF electrode subarrays in the target sequence is controlled by the control module to output RF energy sequentially. In the process of using the RF electrodes to deliver RF energy to the skin treatment area, the treatment area corresponding to each of the RF electrode subarrays is a partial area of the entire treatment area. By performing time-sharing treatment on each partial area, the treatment area in each treatment step is reduced, thereby alleviating the pain in the partial area. At the same time, the control module controls the (N+n)th subarray to output RF energy in an overlapping period with the output period of the Nth subarray, so that the output periods between the two adjacent RF electrode subarrays activated in sequential timings in the target sequence have a partially overlapping period, thereby avoiding the existence of treatment gaps in the working process of the RF electrode array and shortening the overall treatment time. In addition, in the case if the total number of RF electrodes in the RF electrode array is larger, the overall treatment time can be shortened by accumulating energy in the overlapping period, thereby further alleviating the pain reaction in patients.

### BRIEF DESCRIPTION OF THE DRAWINGS

In order to more clearly illustrate the technical solutions in the embodiments of the present application or in the related art, drawings used in the embodiments or in the related art will be briefly described below. Obviously, the drawings in the following description are only some embodiments of the present application. It will be apparent to those skilled in the art that other figures can be obtained according to the structures shown in the drawings without creative work.
FIG. 1 is a schematic diagram of functional modules of a radio frequency (RF) electrode array control device according to an embodiment of the present application.
FIG. 2 is a schematic diagram of the RF electrode array according to an embodiment of the present application.
FIG. 3 is a schematic diagram of a time-period corresponding to an example of an implementation method according to an embodiment of the present application.
FIG. 4 is a schematic diagram of a time-period corresponding to another example of an implementation method according to an embodiment of the present application.
FIG. 5 is a schematic diagram of a time-period corresponding to an example of another implementation method according to an embodiment of the present application.
FIG. 6 is a schematic diagram of the functional modules of an RF treatment equipment according to an embodiment of the present application.

The realization of the purpose, functional features and advantages of the present application will be further described in conjunction with the embodiments, with reference to the accompanying drawings.

### DETAILED DESCRIPTION OF THE EMBODIMENTS

In order to make the purpose, technical solutions and advantages of the present application clearer, the technical solutions in the present application will be clearly and completely described below in conjunction with the accompanying drawings of the present application. Obviously, the described embodiments are part of the embodiments of the present application, not all of the embodiments. Based on the embodiments of the present application, all other embodiments obtained by those of ordinary skill in the art without creative efforts fall within the scope of the present application.

It should be noted that, in this document, the terms "comprising", "comprises" or any other variations thereof are intended to cover a non-exclusive inclusion, such that a process, method, article or system that includes a series of elements not only includes those elements, it also includes other elements not expressly listed or inherent in the process, method, article or system. Without further limitation, an element defined by the statement "comprises a..." does not exclude the presence of additional identical elements in a process, method, article or system that includes that element.

In the present application, unless otherwise clearly stated and limited, the terms "connection", "fixing", etc. should be understood in a broad sense. For example, "fixing" can be a fixed connection, a detachable connection, or an integral body; it can be a mechanical connection or an electrical connection; it can be a direct connection or an indirect connection through an intermediate medium; it can be an internal connection between two elements or an interactive relationship between two elements.

Besides, the descriptions in the present application that refer to "first," "second," etc. are only for descriptive purposes and are not to be interpreted as indicating or implying relative importance or to implicitly indicate the number of technical features indicated. Thus, a feature defined as "first" or "second" may explicitly or implicitly include at least one of the feature. In the present application, suffixes such as "module", "component" or "unit" used to represent elements are used only to facilitate the description of the present application and have no specific meaning in themselves. Therefore, "module", "component" or "unit" can be used in a mixed manner. In addition, technical solutions between the embodiments can be combined with each other, but must be based on the realization of the technical solutions by those skilled in the art, and when the technical solutions are contradictory to each other or cannot be realized, the technical solutions should be considered that the combination does not exist, and the technical solutions are not fallen within the protection scope claimed in the present application.

In the related art, the radio frequency (RF) electrode arrays are classified into minimally invasive and noninvasive types based on whether there is a physical needle available for penetrating the skin to directly deliver high frequency energy into skin dermis (between epidermis and subcutaneous layer). The minimally invasive RF electrode arrays include RF microneedle arrays, which are minimally invasive technologies. By inserting tiny RF microneedles into the epidermis or dermis of the skin, the RF microneedles are used to accurately deliver RF energy to the deep layers of the skin, and the RF energy heats the skin tissue to stimulate the regeneration of skin collagen and dermis, achieving multiple effects such as rejuvenating the skin, tightening pores, and reducing fine lines.

By analysis the related art, the inventors find the problem that the radio frequency electrode array has a larger instantaneous treatment size and a longer treatment time, which may cause bigger pain reaction in patients and poor user experience. In view of the above technical problems, the present application provides an RF electrode array control device and an RF treatment equipment.

The RF electrode array control device and RF treatment equipment provided by the present application are described in detail below through specific embodiments and implementations in conjunction with the accompanying drawings.

### First embodiment

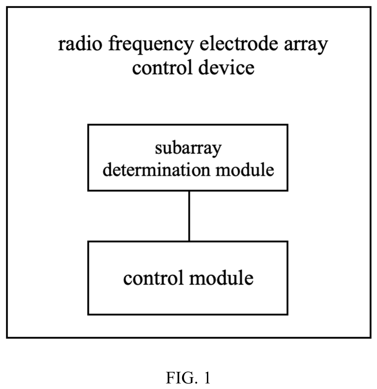

As shown in FIG. 1, an embodiment of the RF electrode array control device of the present application is provided. The RF electrode array control device can be a virtual device, which is applied to an RF treatment equipment. The RF treatment equipment includes an RF electrode array, and the RF electrode array includes multiple RF electrode subarrays. Each of the RF electrode subarrays includes at least one RF electrode.

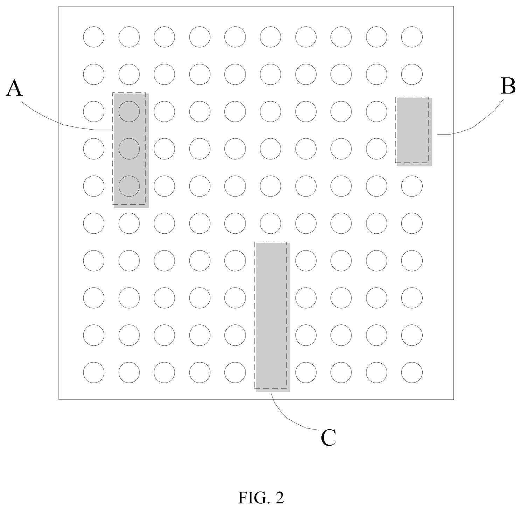

For example, a schematic diagram of an RF electrode array is shown in FIG. 2, in which A, B, and C represent three RF electrode subarrays, respectively. The RF electrode array includes a series of mutually coupled, tiny RF electrodes arranged according to certain rules, and these RF electrodes are usually composed of metal, non-insulating material, and/or insulating material. The RF electrode arrays can be specifically divided into minimally invasive RF electrode arrays and noninvasive RF electrode arrays. For minimally invasive RF electrode arrays, such as RF microneedle arrays, the metal part of the RF electrode is used to transmit RF energy to the deep layer of the skin. The RF electrodes are arranged in the form of metal microneedles and can penetrate deep into the skin. This arrangement of RF electrodes can ensure uniform and precise RF energy delivery to the skin during treatment, and stimulate collagen production and skin regeneration and reconstruction through physical microneedle puncture and RF energy delivery. For noninvasive RF electrode arrays, the RF electrodes are placed close to the skin surface, and the metal part of the RF electrode is used to transmit RF energy to the surface of the skin. The RF electrodes are arranged in the form of metal electrode sheets and can contact the surface of the skin through a thin film. This arrangement of RF electrodes can ensure uniform and precise RF energy delivery to the surface of the skin during treatment, and promote new cell fiber synthesis and skin firming repair through the patch of the metal electrode sheet and the transmission of RF energy. It is worth mentioning that the RF electrode array can be provided at different positions of the RF treatment equipment, and its specific position depends on the device design. Usually, the RF electrode array is provided at the treatment end of the handle of the RF treatment equipment.

For each RF electrode subarray, the polarities of the RF electrodes contained therein may be completely the same or may not be completely the same. The polarities of the RF electrodes may be specifically set according to actual conditions.

As shown in FIG. 1, which is a schematic diagram of functional modules of the RF electrode array control device. The RF electrode array control device may include a subarray determination module and a control module. The RF electrode array control device provided in this embodiment is described in detail below in conjunction with the schematic diagram of the functional modules shown in FIG. 1.

The subarray determination module is used to determine a target time sequence based on the RF electrode array, the target time sequence at least including the Nth subarray and the (N+n)th subarray activated in sequential timings, and N and n are both positive integers.

In this embodiment, relevant personnel can divide all the RF electrodes contained in the RF electrode array in advance or in real time to obtain multiple RF electrode subarrays, and then generate a target sequence. As an embodiment, when determining the RF electrode subarray, the spatial positions of the RF electrodes contained in the RF electrode subarray are adjacent, so as to quickly obtain multiple RF electrode subarrays. In this embodiment, n=1, and the Nth subarray and the (N+n)th subarray are RF electrode subarrays successively activated in the target time sequence selected in the target sequence.

In an embodiment, the subarray determination module includes a first subarray determination submodule, a second subarray determination submodule and a target sequence generation submodule, which are described in detail in turn as follows.

The first subarray determination submodule is used to determine the first RF electrode subarray for outputting RF energy after the RF electrode array is activated (i.e., the first activated RF electrode subarray) from the RF electrode array, and store the first RF electrode subarray in the initial sequence as the target RF electrode subarray.

Before executing the first subarray determination submodule, the initial sequence is empty, that is, there is no element in the initial sequence, the length of the initial sequence is zero, and the first RF electrode subarray for outputting RF energy after the RF electrode array is activated is determined from the RF electrode array. The target RF electrode subarray is the first element in the initial sequence.

When determining the first RF electrode subarray to output RF energy after the RF electrode array is activated, all RF electrode subarrays are available for selection, that is, they are all RF electrode subarrays to be selected. In this embodiment, a random selection method is used to determine the first RF electrode subarray to output RF energy after the RF electrode array is activated from the RF electrode array. The first subarray determination submodule can also use other methods to determine the first RF electrode subarray to output RF energy after the RF electrode array is activated, such as a look-up table. This application does not limit the method for determining the target RF electrode subarray.

The second subarray determination submodule is used to determine a new target RF electrode subarray from the remaining RF electrode subarrays in the RF electrode array excluding target RF electrode subarrays, and store the new target RF electrode subarray in the initial sequence.

During a treatment process, the RF energy received by the skin area acted upon by the RF electrode array only needs to reach a certain value. Based on this, when determining the RF electrode subarray that subsequently outputs RF energy, i.e., the new target RF electrode subarray, it is selected from the remaining RF electrode subarrays in the RF electrode array except for all historical RF electrode subarrays. This can avoid excessive RF energy being delivered to the same skin area, which may cause adverse effects on the human body.

The target sequence generation submodule is used to repeat executing the second subarray determination submodule until all RF electrode subarrays in the RF electrode array are target RF electrode subarrays, thereby obtaining a target sequence.

The second subarray determination submodule may be directly connected to the first subarray determination submodule, and automatically repeat executing after the first subarray determination submodule executes an action. The second subarray determination submodule may also be connected to the control module, and the first subarray determination submodule and the second subarray determination submodule may be controlled by the control module respectively, the second subarray determination submodule may be executed after the first subarray determination submodule is executed, and the second subarray determination submodule may be executed repeatedly.

In this embodiment, after the first subarray determination submodule obtains the first RF electrode subarray for outputting RF energy after the RF electrode array is activated, that is, the target RF electrode subarray. The second subarray determination submodule determines the second RF electrode subarray for outputting RF energy after the RF electrode array is activated, that is, the new target RF electrode subarray. The target sequence generation submodule then controls the second subarray determination submodule to execute in a loop until a plurality of target RF electrode subarrays are determined in the loop, and ensures that all RF electrode subarrays in the RF electrode array are target RF electrode subarrays, thereby obtaining the target sequence.

In an embodiment, the Nth subarray and the (N+1)th subarray are RF electrode subarrays successively activated in the target time sequence in the target sequence. The Nth subarray and the (N+1)th subarray are not physically adjacent.

Correspondingly, the second subarray determination submodule is specifically used to: determine a screening subarray set from the remaining RF electrode subarrays excluding target RF electrode subarrays in the RF electrode array, determine a new target RF electrode subarray based on the screening subarray set, and store the new target RF electrode subarray in the initial sequence. The screening subarray set does not include the RF electrode subarray that is physically adjacent to the last target RF electrode subarray in the current initial sequence. The result presented by this manner is that for the optional Nth subarray and the (N+1)th subarray that are adjacent in activation sequence, the two groups of subarrays are not adjacent in spatial position.

The RF energy output by each RF electrode subarray acts only on a part of the skin area to be treated. By determining all RF electrode subarrays that are not adjacent to the last target RF electrode subarray in the current initial sequence from the remaining RF electrode subarrays excluding target RF electrode subarrays in the RF electrode array, and then determining a new target RF electrode subarray from all RF electrode subarrays that are not adjacent to the last target RF electrode subarray in the current initial sequence, it is possible to avoid obvious pain in the local skin caused by the skin areas acted on by the two RF electrode subarrays that output RF energy one after another being close.

In another embodiment, the second subarray determination submodule is specifically configured to: randomly select an RF electrode subarray from the remaining RF electrode subarrays excluding target RF electrode subarrays in the RF electrode array as a new target RF electrode subarray, and store the new target RF electrode subarray in the initial sequence.

The time period of traversing and activating all RF electrode subarrays in the target sequence and completing one output period can be regarded as an overall time period. Different overall time periods may correspond to different target sequences. Different target sequences refer to that the activation order (order of outputting RF energy) of the RF electrode subarrays in each target sequence may be different. Therefore, when determining a new target RF electrode subarray, the second subarray determination submodule can randomly select from the remaining electrode subarrays to generate different target sequences. In the present application, for the same target treatment area, it can be controlled to perform only one overall time period of treatment, or it can be controlled to perform more than one overall time period of treatment.

After all the determined target RF electrode subarrays are obtained, the target RF electrode subarrays that output RF energy after the last RF electrode subarray in the current initial sequence can also be randomly generated to ensure that too much or too little RF energy is not delivered to the same skin area. As a presentation of the method, in different target sequences, the RF output order, i.e., the activation timing, of the RF electrode subarrays may be different.

Regardless of the embodiment method selected by the second subarray determination submodule, the target sequence obtained by the target sequence generation submodule follows a certain activation timing, that is, the first element in the target sequence is the first RF electrode subarray for outputting RF energy after the RF electrode array is activated, and the last element is the last RF electrode subarray for outputting RF energy after the RF electrode array is activated.

After the first subarray determination submodule determines the first RF electrode subarray for outputting RF energy after the RF electrode array is activated and uses it as the target RF electrode subarray, the target sequence generation submodule can directly determine the target RF electrode subarrays corresponding to multiple output periods at one time based on the remaining RF electrode subarrays in the RF electrode array through a period, and finally realize that each RF electrode subarray outputs RF energy in its corresponding output period. The target sequence generation submodule can also determine the RF electrode subarray that has an overlapping period with the output period of the RF electrode subarray that currently outputs RF energy during the actual operation of the RF electrode array, before the start of the output period corresponding to the RF electrode subarray that currently outputs RF energy or the start of the overlapping period corresponding to the output period, that is, determine the N+1 subarray before the start of the output period of the Nth subarray or the corresponding overlapping period, thereby obtaining multiple target RF electrode subarrays to generate a target sequence. The output period of the RF electrode subarray refers to the time period in which the RF electrode subarray outputs RF energy within an overall time period. The overlapping period corresponding to the output period of the Nth subarray refers to the time period in which the output period of the Nth subarray overlaps with the output period of the (N+1)th subarray.

In this embodiment, all target RF electrode subarrays are determined based on the target sequence generation submodule, so that each RF electrode subarray outputs RF energy in its corresponding output period, thereby completing the treatment of all areas included in the skin area and achieving the purpose of treatment.

The RF electrode array control device provided in this embodiment will be described in detail below in conjunction with the schematic diagram of the functional module shown in FIG. 1.

The control module is used to control each RF electrode subarray in the target sequence to output RF energy in sequence, and to control the (N+n)th subarray to output RF energy in an overlapping period corresponding to the output period of the Nth subarray.

That is, in the target sequence, there are at least two RF electrode subarrays with successive activation timing, and there is an overlapping period in which they output RF energy together. According to the activation sequence, these two RF electrode subarrays are the Nth subarray and the (N+n)th subarray, respectively, where N and n are both positive integers.

The following is a detailed description of an embodiment in which n=1 is taken. In an embodiment, the RF electrode subarray currently outputting RF energy is taken as the first subarray, and the RF electrode subarray located after and adjacent to the first subarray in the target sequence is taken as the second subarray. That is, the first subarray and the second subarray are two adjacent RF electrode subarrays activated in sequential timings. In this case, the control module can control the second subarray to output RF energy within the overlapping period corresponding to the output period of the first subarray.

The output periods corresponding to the RF electrode subarrays is provided with a sequence, and each output period has its own start time and end time. For each RF electrode subarray excluding the RF electrode subarray corresponding to the last target RF electrode subarray in the target sequence, its output period has a partially overlapping time period with the output period of the next RF electrode subarray adjacent to the activation sequence. That is, there is a partially overlapping time period between the output period corresponding to the Nth subarray and the output period corresponding to the (N+1)th subarray, that is, the overlapping time period, and the duration and start time of the overlapping time period can be set according to actual needs.

In an embodiment, the Nth subarray and the (N+1)th subarray are RF electrode subarrays successively activated in the target time sequence in an optional manner in a target sequence.

For multiple RF electrode subarrays in the target sequence, there may be an overlapping period between two RF electrode subarrays successively activated in the target time sequence. There may be an overlapping period between two RF electrode subarrays successively activated in the target time sequence at any time, that is, the Nth subarray and the (N+1)th subarray are RF electrode subarrays successively activated in the target time sequence at any time in the target sequence. When there is an overlapping period between two RF electrode subarrays successively activated in the target time sequence at any time, the overall action time of the RF electrode array can be shortened further.

In an embodiment, within an overall time period of activating all RF electrode subarrays, the output period of each RF electrode subarray is continuous without interruption.

In an embodiment, when the Nth subarray and the (N+1)th subarray are RF electrode subarrays successively activated in the target time sequence in the target sequence, for the RF electrode subarrays (Nth subarray) excluding the last target RF electrode subarray in the target sequence, there is a preset overlapping time period, i.e., an overlapping time period, between the output period corresponding to the RF electrode subarray and the output period corresponding to the next RF electrode subarray ((N+1)th subarray). The number of the overlapping time periods is 1, and the end time of the overlapping time period is the end time of the output period corresponding to the Nth subarray, so that the time period in which the RF electrode subarray ((N+1)th subarray) located after and adjacent to the RF electrode subarray in the target sequence outputs RF energy is continuous without interruption. The result presented by this embodiment is that the output period of any RF electrode subarray in the target sequence is continuous without interruption and there is an overlapping time period for any two RF electrode subarrays successively activated in the target time sequence.

In an embodiment, in the target sequence, the output period of the Nth subarray includes an overlapping period with the output period of the (N-1)th subarray and an overlapping period with the output period of the (N+1)th subarray, where N is a positive integer other than 1, and the maximum value of N is less than the total number of RF electrode subarrays in the RF electrode array.

In an embodiment, for each RF electrode subarray exculsive of the last RF electrode subarray in the target sequence, the overlapping period with the output period of the RF electrode subarray adjacent to the next activation sequence can be preset as a continuous partial period of the output period, that is, the overlapping period can be a certain proportion of the output period, such as 30%, 40%, etc. of the output period or half of the output period. The proportion can be less than or equal to 50%, so that in any overlapping period of the overall time period, at most only 2 RF electrode subarrays are in the output period, that is, at most only 2 RF electrode subarrays output RF energy at the same time.

In an embodiment, the start time of the overlap period corresponding to the output period of the Nth subarray and the output period of the (N+1)th subarray is preset as the middle time of the output period of the Nth subarray, and the end time of the overlap period is the end time of the output period. The result presented by this embodiment is that in the target sequence, except for the first RF electrode subarray and the last RF electrode subarray, the output periods of other RF electrode subarrays are all within the overlap period.

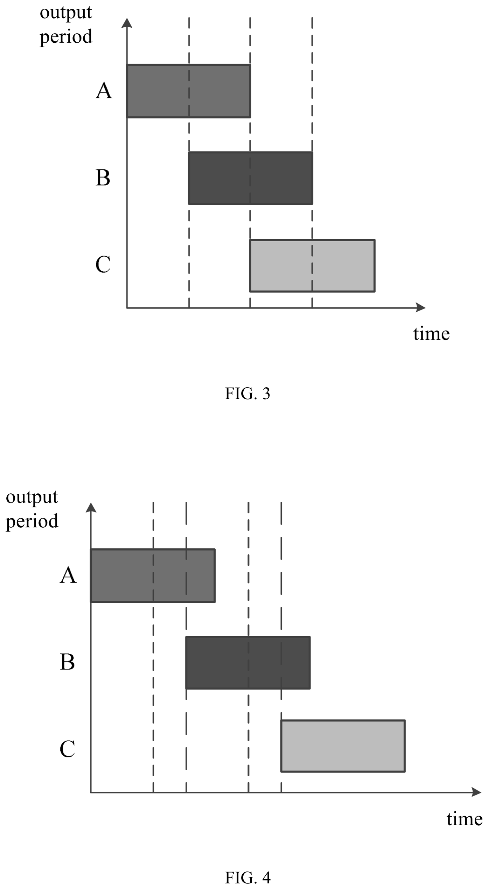

As shown in FIG. 3, it is a schematic diagram of the time-period of this example, which shows the relationship between the continuous multiple output period times and the working time of the RF electrode array when the overlapping period is set as a continuous half period in the output period and the starting time of the overlapping period is the middle time of the output period. In FIG. 3, A, B, and C respectively represent three continuous output periods, i.e., the output periods of three RF electrode subarrays successively activated in the target time sequence. According to FIG. 3, it can be seen that for each RF electrode subarray excluding the last RF electrode subarray in the target sequence, by setting the overlapping period corresponding to its output period and the output period of the RF electrode subarray of the next activation sequence as a continuous half period in the output period, the starting time of the overlapping period is the middle time of the output period. In this case, the output period of each RF electrode subarray excluding the first RF electrode subarray and the last RF electrode subarray in the target sequence is composed only of the overlapping period corresponding to the output period of the RF electrode subarray adjacent to the previous activation sequence and the overlapping period corresponding to the output period of the RF electrode subarray adjacent to the next activation sequence, and these two overlapping periods are continuous. This manner can make the time period during which each RF electrode subarray outputs RF energy completely continuous. When each RF electrode subarray excluding the first and the last RF electrode subarray in the target sequence outputs RF energy, there is no time period outside the non-overlapping time period, that is, the output periods of other RF electrode subarrays are all within the overlapping time period. This manner can significantly reduce the treatment time, that is, reduce the action time of the RF electrode array, and reduce the user's pain overall.

In an embodiment, in the target sequence, at least part of the output period of one RF electrode subarray from the RF electrode subarrays include the part during which only the one RF electrode subarray outputs RF energy.

In an embodiment, for each RF electrode subarray excluding the last RF electrode subarray in the target sequence, the duration of the overlapping period corresponding to the output period of its output period and the output period of the RF electrode subarray adjacent to the next activation sequence can be preset to be less than the duration of half the output period. That is, in this case, the starting time of the overlapping period corresponding to the output period of the Nth subarray and the output period of the (N+1)th subarray can be preset to be the time corresponding to the end time of the output period of the Nth subarray minus the duration of the overlapping period. The result presented by this embodiment is that, in the target sequence, except for the first RF electrode subarray and the last RF electrode subarray, at least some other RF electrode subarrays have non-overlapping time periods, that is, only the time period when the RF electrode subarray outputs RF energy.

As shown in FIG. 4, it is a schematic diagram of a time-period, which shows a relationship between multiple consecutive output period times and the working time of the RF electrode array when the overlapping period is set to a time period whose duration is less than half a period of the output period, and the starting time of the overlapping period is the time corresponding to the end time of the output period minus the duration of the overlapping period. In FIG. 4, A, B, and C respectively represent three consecutive output periods, i.e., the output periods of three RF electrode subarrays successively activated in the target time sequences. According to FIG. 4, it can be seen that for each RF electrode subarray in the target sequence excluding the last RF electrode subarray, the duration of the overlapping period corresponding to its output period and the output period of the RF electrode subarray of the next activation sequence is set to a time period less than half a period of the output period. At this time, the starting time of the overlapping period is the end time of the output period minus the time corresponding to the length of the overlapping period. In this case, the output period of each RF electrode subarray in the target sequence excluding the first RF electrode subarray and the last RF electrode subarray is composed of the overlapping period corresponding to the output period of the RF electrode subarray adjacent to the previous activation sequence, the time period when only the RF electrode subarray outputs RF energy itself, and the overlapping period corresponding to the output period of the RF electrode subarray adjacent to the next activation sequence, and these three time periods are continuous. This manner can make the time period during which each RF electrode subarray outputs RF energy completely continuous. Except for the first RF electrode subarray and the last RF electrode subarray in the target sequence, when each RF electrode subarray outputs RF energy, there are continuous overlapping time periods and non-overlapping time periods, where the non-overlapping time period, that is, the output period of each RF electrode subarray, has at least one time period during which only the RF electrode subarray outputs RF energy. By overlapping the action time of the RF electrode subarrays, the action time of the RF electrode arrays is reduced, and the user's pain is reduced as a whole.

In another embodiment, within an overall time period of traversing and activating all RF electrode subarrays, the output period of any RF electrode subarray is a noncontinuous time period.

In an embodiment, for any RF electrode subarray excluding the first RF electrode subarray in the target sequence, the number of time periods corresponding to its output period is at least 2 and the at least 2 time periods are noncontinuous, and/or the number of overlapping time periods corresponding to the output period of the RF electrode subarray adjacent to the subsequent activation sequence is 1 and the end time of the overlapping time period is not the end time of the output period of any RF electrode subarray, so that the time period in which any RF electrode subarray excluding the first RF electrode subarray and the last RF electrode subarray in the target sequence outputs RF energy is a noncontinuous time period.

In an embodiment, within an overall time period of traversing and activating all RF electrode subarrays, the output period of the (N+n)th subarray includes at least two noncontinuous RF energy output time periods. For the at least two noncontinuous RF energy output time periods, in an embodiment, the preceding RF energy output time period overlaps with the output period of the Nth subarray. In an embodiment, the succeeding RF energy output time period overlaps with the output period of the Nth subarray.

N is a positive integer, and the maximum value of N is less than the total number of RF electrode subarrays in the RF electrode array, that is, the (N+1)th subarray may be the last RF electrode subarray in the target sequence.

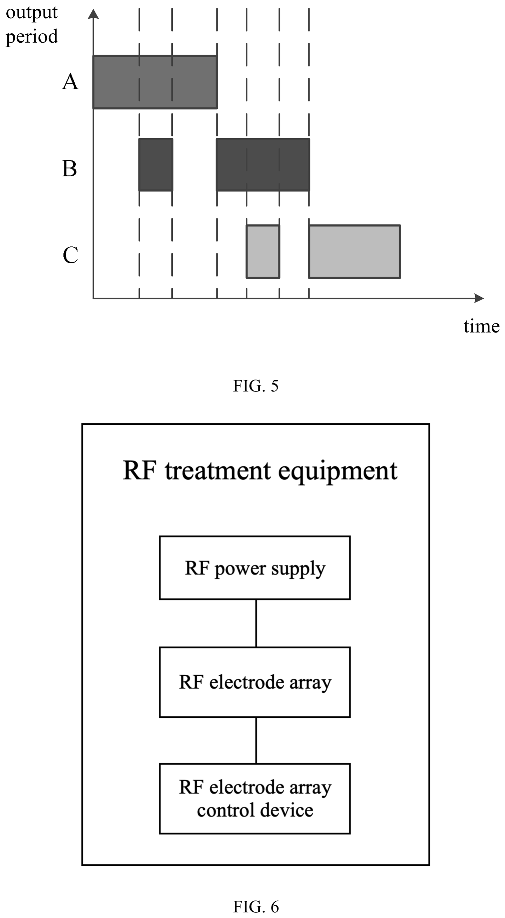

As shown in FIG. 5, it is a time-period diagram of this embodiment, which shows the relationship between multiple consecutive output period times and the working time of the RF electrode array when the overlapping time period is set to 1 and the end time is not the end time of the output period of the RF electrode subarray. In FIG. 5, A, B, and C respectively represent three consecutive output periods, that is, the output periods of three RF electrode subarrays successively activated in the target time sequence. According to FIG. 5, for each RF electrode subarray excluding the last RF electrode subarray in the target sequence, by setting the overlapping time period corresponding to its output period to 1 and the end time of the overlapping time period not being the end time of its output period, the time for all RF electrode subarrays (any (N+1)th subarray ) excluding the first RF electrode subarray (1st subarray) for outputting RF energy after the RF electrode array is activated is not completely continuous, and each RF electrode subarray excluding the first RF electrode subarray and the last RF electrode subarray in the target sequence has a non-continuous overlapping time period with the two RF electrode subarrays before and after it that are adjacent in activation sequence in the target sequence. That is, in FIG. 5, the output period of any (N+1)th subarray includes two noncontinuous RF energy output time periods, the former RF energy output time period has an overlapping time period with the output period of the Nth subarray, and the latter RF energy output time period has an overlapping time period with the output period of the (N+1)th subarray. This manner can also reduce the duration of the action of the RF electrode array and reduce the user's pain as a whole.

Based on FIG. 3 to FIG. 5, it can be seen that in any overlapping period, the number of RF electrode subarrays in the output period is 2, that is, the number of RF electrode subarrays outputting RF energy at any time is 2.

For each RF electrode subarray excluding the last target RF electrode subarray in the target sequence, an overlapping period is set so that the time periods of the RF energy output by two adjacent RF electrode subarrays activated in sequential timings in the target sequence overlap, thereby avoiding treatment gaps during the operation of the RF electrode array (i.e. avoiding the existence of stop time periods within an overall time period of the RF energy output by the RF electrode array). Moreover, when the RF electrode array includes a large number of RF electrodes, the number of time-sharing treatments of the RF electrode array can be reduced, thereby shortening the time required to complete a treatment and reducing the overall treatment time. At the same time, by reducing the skin area acted on at the same time, the patient's perception of and pain from RF energy can be reduced.

Of course, when only the last RF electrode subarray in the target sequence outputs RF energy, it indicates that the current output period is the last output period to complete this treatment. At this time, all the RF electrode subarrays excluding the last RF electrode subarray in the target sequence have completed the treatment of the skin area they act on.

Next, in combination with the description of the above embodiment of n=1, the embodiment of n>1 in the present application is briefly described.

In the embodiment where n>1, the control module controls the (N+n)th subarray to output RF energy in the overlapping period corresponding to the output period of the Nth subarray. That is, in the target sequence generated by the subarray determination module, the Nth subarray and the (N+n)th subarray, which are activated one after the other but not adjacent, have a common overlapping period. This embodiment can also use the overlapping period to accumulate energy, shorten the overall action time, and reduce the user's pain, as in the above-mentioned embodiment where n=1.

In the embodiment where n>1, if the output period of each RF electrode subarray within the overall time period is continuous without interruption, then the Nth, N+1..., (N+n)th subarrays must have a common overlapping period. In order to avoid excessive number of RF electrode subarrays in the output period at the same time and causing severe pain, the present application provides the number of RF electrode subarrays in the output period at the same time to be less than a preset value, which can be, for example, 3 or 4.

In the embodiment where n>1, if there is an RF electrode subarray whose output period is noncontinuous within an overall time period, there may be an RF electrode subarray between the Nth subarray and the (N+n)th subarray that does not overlap with the Nth subarray. For example, the output period of the first subarray is divided into two noncontinuous RF energy output time periods (referred to as output time periods), the output period of the second subarray does not overlap with the output period of the first subarray, and is at least partially located between the two output time periods of the first subarray, and the output period of the third subarray overlaps with the subsequent output time period of the first subarray.

Regarding the embodiment of n>1, other technical solutions that do not conflict with the embodiment of n=1 can refer to the above description, such as the function and structure of the subarray determination module, and will not be repeated here.

It needs to be emphasized again that the pain generated by the RF electrode array is closely related to the area of RF energy output at the same time. Regardless of whether n=1 or n>1, in some embodiments of the present application, a certain RF electrode subarray may also have overlapping periods with more than one other RF electrode subarray. Regardless of the number of RF electrode subarrays in the overlapping period, in any overlapping period, the RF electrode subarrays in the output period at the same time are not adjacent to each other in space to avoid obvious pain in the local skin.

In an embodiment, the RF electrode array control device provided in this embodiment further includes an output accumulating module. The output accumulating module is used to count the accumulated RF energy value outputted by each RF electrode subarray from the first activation moment to the current moment, and the control module is also used to control the RF electrode subarray to be in a power-free output state if the accumulated RF energy value of the RF electrode subarray is greater than or equal to a preset threshold for each RF electrode subarray.

The historical output period refers to the output period corresponding to the RF electrode subarray that has output RF energy. For each RF electrode subarray, this embodiment counts the accumulated RF energy value that has been output by the RF electrode subarray from the first activation moment to the current moment, and controls the RF electrode subarray to be in a power-free output state according to a preset threshold, thereby avoiding excessive RF energy output to the same skin area of the human body and causing pain to the human body.

In the RF electrode array control device provided in this embodiment, the control module further includes a temperature parameter acquisition unit and an output power adjustment unit.

The temperature parameter acquisition unit is used to acquire the real-time temperature parameters collected by the temperature sensor. The output power adjustment unit is used to adjust the real-time output power of the RF electrode subarray that currently outputs RF energy according to the real-time temperature parameters.

One or more temperature sensors may be provided in the RF treatment equipment to detect the temperature changes of the skin surface and deep tissue. In addition, when the RF electrode subarray currently outputting RF energy outputs RF energy at a fixed power, the temperature of the affected skin area will rise, and when the human skin temperature rises, it may cause pain.

Based on this, in an embodiment, the output power adjustment unit is specifically used to: when the real-time temperature parameter is less than the preset temperature threshold, allow the control module to control the RF electrode subarray that is currently outputting RF energy to output RF energy with a preset power. The real-time temperature parameter refers to the temperature parameter of the skin area acted upon by the RF electrode subarray that is currently outputting RF energy. When the real-time temperature parameter is greater than or equal to the preset temperature threshold, the output power of the RF electrode subarray that is currently outputting RF energy is adjusted, and the control module controls the RF electrode subarray to output RF energy with the adjusted output power, so that the temperature of the skin area acted upon by the RF electrode subarray that is currently outputting RF energy does not exceed the preset temperature threshold, and the real-time output power is less than the preset power.

This embodiment acquires the real-time temperature parameters collected by the temperature sensor and controls the temperature of the skin area acted upon by the RF electrode subarray that currently outputs RF energy not to exceed a preset temperature threshold. That is, when the RF electrode subarray outputs RF energy, the temperature of the skin area acted upon first rises and then remains constant, thereby avoiding pain in the human body.

In an embodiment, the RF electrode subarray includes at least two RF electrodes that are not adjacent to each other.

For example, if the three RF electrodes at A and the two RF electrodes at B in FIG. 2 are determined as the first subarray of the target sequence, then the subsequent control of the first subarray is actually the control of the five RF electrodes at the same time, that is, the multiple RF electrodes that are not adjacent in spatial position are regarded as a whole, as an RF electrode subarray of a certain activation sequence in the target sequence. By determining that multiple RF electrodes that are not adjacent in spatial position are an RF electrode subarray, the number of times the target RF electrode subarray is determined from the RF electrode array during treatment can be reduced. That is, the number of output periods is reduced, thereby reducing the time required to complete a treatment. Moreover, when the total treatment area is the same, the total treatment area composed of multiple non-adjacent treatment areas (i.e., the action areas corresponding to the RF electrodes) causes less pain to the human body than the total treatment area composed of adjacent treatment areas.

In an embodiment, in the RF electrode array control device provided in this embodiment, each of the RF electrode subarray includes an identical number of RF electrodes, or the difference between numbers of the RF electrodes of any two RF electrode subarrays is less than a preset number threshold, so as to control the uniformity of treatment energy distribution.

The RF electrode array control device provided in the present embodiment includes a subarray determination module and a control module. The subarray determination module generates a target sequence according to a plurality of RF electrode subarrays of the RF electrode array, and then the control module controls each RF electrode subarray in the target sequence to output RF energy in sequence. In the process of using RF electrodes to deliver RF energy to the skin action area, the action area corresponding to each RF electrode subarray is a local area of the entire treatment area. By performing time-sharing treatment on each local area, the instantaneous treatment area at each moment is reduced, thereby alleviating the pain in the local area. At the same time, the control module controls the (N+1)th subarray to output RF energy in the overlapping time period corresponding to the output period of the Nth subarray, so that the output periods corresponding to the two adjacent RF electrode subarrays activated in sequential timings in the target sequence have a partially overlapping time period, thereby avoiding the existence of treatment gaps in the working process of the RF electrode array and causing the overall action time to increase. Moreover, in the case where the total number of RF electrodes in the RF electrode array is large, the overall action time can be shortened by accumulating energy in the overlapping time period, thereby further alleviating the user's pain.

When the RF electrode array control device provided in this embodiment is applied to an RF treatment equipment, the RF treatment equipment includes an RF generator and an RF electrode array. The RF electrode array is electrically connected to the RF generator. The RF electrode array includes multiple RF electrode subarrays, and each RF electrode subarray includes at least one RF electrode.

In an embodiment, only one RF generator may be provided, that is, a single RF generator powers the RF electrode array, or multiple RF generator may be provided, that is, multiple RF generators are respectively connected to the switch module of the RF treatment equipment, and the switch module is controlled by the control unit of the RF treatment equipment to achieve connection and disconnection between the RF electrode array and the RF generator to adjust the output and transmission of RF energy.

### Second embodiment

Based on the same inventive concept, as shown in FIG. 6, the present application further provides an RF treatment equipment. The RF treatment equipment of the present embodiment is described in detail below in conjunction with the schematic diagram of the functional module shown in FIG. 6.

The RF treatment equipment includes an RF generator, an RF electrode array and a RF electrode array control device as in the first embodiment, and both the RF generator and the RF electrode array control device are connected to the RF electrode array.

In an embodiment, the RF treatment equipment includes an RF generator, and each RF electrode subarray is connected in parallel to the RF generator, and the RF generator generates unchanged output power during the overlapping period.

When the RF treatment equipment includes only one RF generator, all RF electrode subarrays included in the RF electrode array are powered by the RF generator. In this embodiment, for each RF electrode subarray (the Nth subarray) excluding the last RF electrode subarray in the target sequence, when the time period during which the RF electrode subarray outputs RF energy is an overlapping period corresponding to its output period, the RF electrode subarray and another RF electrode subarray (the (N+1)th subarray) whose activation timing is subsequent to and adjacent to the subarray in the target sequence are connected in parallel for power distribution.

In an embodiment, for each RF electrode subarray excluding the last RF electrode subarray in the target sequence, if the total power output by the RF generator is a fixed value, the RF generator needs to output power to the Nth subarray and the (N+1)th subarray, so that the average power of the Nth subarray in the overlapping time period corresponding to its output period is less than the average power in other time periods in the output period excluding the overlapping time period. If the total power output by the RF generator is a variable value, the total output power of the RF electrode array can be increased during the overlapping time period corresponding to the output period of the RF electrode subarray controlled by the RF electrode array control device.

In an embodiment, the RF generator includes a plurality of sub-RF power supplies, each of which is connected with each of the RF electrode subarrays to allow an output power of each RF electrode subarray to remain unchanged.

When the RF treatment equipment includes multiple sub-RF power supplies, different RF electrode subarrays can be controlled separately by different sub-RF power supplies, that is, they are independently controlled by the corresponding sub-RF power supplies. Of course, one sub-RF power supply can also control multiple RF electrode subarrays. For example, when there are twenty RF electrode subarrays and five sub-RF power supplies, each sub-RF power supply can control four RF electrode subarrays. In an embodiment, for each RF electrode subarray (the Nth subarray) excluding the last RF electrode subarray in the target sequence, the sub-RF power supply connected to the RF electrode subarray is different from the sub-RF power supply connected to another RF electrode subarray (the (N+1)th subarray) whose activation timing is subsequent to it and adjacent to it in the target sequence, thereby avoiding the average power drop of the RF electrode subarray during the overlapping period corresponding to its output period, thereby shortening the treatment time.

In an embodiment, the RF treatment equipment includes a monopolar mode, in which polarities of RF electrodes of any one RF electrode subarray are the same, while an electrode plate of the RF treatment equipment has an opposite polarity.

In an embodiment, when the working mode of the RF treatment equipment is monopolar mode, for each RF electrode subarray, the polarity of the needle electrodes of all the RF electrodes contained therein is the same, and the non-needle electrodes of the other polarity can be attached as a whole to the back, buttocks and other positions of the human body.

In an embodiment, the RF treatment equipment includes a bipolar mode. Each of the RF electrode subarrays includes at least two RF electrodes with opposite polarities in the bipolar mode.

In an embodiment, the polarity of the needle electrodes of the RF electrodes have both positive polarity and negative polarity, and the RF electrodes with positive polarity and the RF electrodes with negative polarity can together constitute an RF electrode subarray.

Better treatment effects can be achieved by selecting the appropriate working mode based on the patient's specific condition and treatment needs.

The serial numbers of the embodiments of the present application are for description only and do not represent the advantages and disadvantages of the embodiments. The above are only some embodiments of the present application, and are not intended to limit the scope of the present application. Any equivalent structures or equivalent process transformations made using the contents of the description and the accompanying drawings of the present application, or direct or indirect applications in other related technical fields, are all included in the scope of the present application.

## Claims

1. A radio frequency (RF) electrode array control device, applied to an RF treatment equipment, wherein the RF treatment equipment comprises an RF electrode array, the RF electrode array comprises a plurality of RF electrode subarrays, and each of the RF electrode subarrays comprises at least one RF electrode, the control device **characterized by** comprising:
a subarray determination module, configured to determine a target time sequence based on the RF electrode array, the target time sequence at least including a Nth subarray and a (N+n)th subarray activated in sequential timings, wherein N and n are both positive integers; and
a control module, configured to control the RF electrode subarrays in the target time sequence to be individually activated to output RF energy, and control the Nth subarray and the (N+n)th subarray simultaneously output RF energy in an overlapping period between an output period of the Nth subarray and the output period of the (N+n)th subarray.

2. The RF electrode array control device of claim 1, wherein in response to determining n is one, the Nth subarray and the (N+n)th subarray are successively activated in the target time sequence.

3. The RF electrode array control device of claim 1, wherein in an overall time period during which the RF electrode subarrays are controlled to be individually activated, the output period of each RF electrode subarray is continuous without interruption.

4. The RF electrode array control device of claim 3, wherein at least part of the output periods of the RF electrode subarrays in the target time sequence comprises a first time period during which only one RF electrode subarray is activated to output RF energy.

5. The RF electrode array control device of claim 1, wherein in an overall time period during which the RF electrode subarrays are controlled to be individually activated, the output period of the (N+n)th subarray comprises at least two noncontinuous second time periods during which the (N+n)th subarray is activated to output RF energy.

6. The RF electrode array control device of claim 1, wherein the subarray determination module comprises:
a first subarray determination submodule, configured to determine a first RF electrode subarray is activated to output RF energy, and store the first RF electrode subarray as a target RF electrode subarray in an initial sequence;
a second subarray determination submodule, configured to determine a new target RF electrode subarray from the remaining RF electrode subarrays in the RF electrode array excluding the target RF electrode subarrays, and store the new target RF electrode subarray in the initial sequence; and
a target sequence generation submodule, configured to repeat executing the second subarray determination submodule to obtain the target time sequence until all RF electrode subarrays in the RF electrode array are the target RF electrode subarrays.

7. The RF electrode array control device of claim 6, wherein in any of the overlapping periods between the output periods of the Nth subarray and the output period of the (N+n)th subarray, the Nth subarrays and the (N+n)th subarray are not physically adjacent with each other.

8. The RF electrode array control device of claim 6, wherein the second subarray determination submodule is configured to:
randomly select the RF electrode subarray from the remaining RF electrode subarrays in the RF electrode array excluding the target RF electrode subarrays as a new target RF electrode subarray, and store the new target RF electrode subarray in the initial sequence.

9. The RF electrode array control device of any one of claims 1 to 8, further comprising an output accumulating module, configured to accumulate the RF energy outputted by each of the RF electrode subarrays activated from firstly to currently; and
wherein when activating each of the RF electrode subarrays, in response to determining the accumulated RF energy of a certain RF electrode subarray is greater than or equal to a preset threshold, and the control module further controls the RF electrode subarray to stop outputting the RF energy.

10. The RF electrode array control device of any one of claims 1 to 8, wherein the control module further comprises:
a temperature parameter acquisition unit, configured to acquire a real-time temperature parameter collected by a temperature sensor; and
an output power adjustment unit, configured to adjust a real-time output power of an RF electrode subarray currently outputting RF energy according to the real-time temperature parameter.

11. The RF electrode array control device of any one of claims 1 to 8, wherein each of the RF electrode subarrays comprises an identical number of RF electrodes, or a difference between numbers of the RF electrodes of any two RF electrode subarrays is less than a preset number threshold.

12. A radio frequency (RF) treatment equipment, **characterized by** comprising:
an RF power supply;
an RF electrode array; and
the RF electrode array control device of any one of claims 1 to 11, wherein the RF power supply and the RF electrode array control device are both connected to the RF electrode array.

13. The RF treatment equipment of claim 12, wherein each of the RF electrode subarrays is connected in parallel and connected to the RF power supply, wherein the RF power supply generates unchanged output power during the overlapping period between the output periods of the Nth subarray and the (N+n)th subarray.

14. The RF treatment equipment of claim 12, wherein the RF power supply comprises a plurality of sub-RF power supplies, each of which is connected with each of the RF electrode subarrays to allow an output power of each RF electrode subarray to remain unchanged.

15. The RF treatment equipment of claim 12, further comprising a monopolar mode, polarities of RF electrodes of any one RF electrode subarray are same, while an electrode plate of the RF treatment equipment has an opposite polarity.

16. The RF treatment equipment of claim 12, further comprising a bipolar mode, each of the RF electrode subarrays comprises at least two RF electrodes with opposite polarities in the bipolar mode.
